**Europäisches Patentamt**

**European Patent Office**

**Office européen des brevets**

(11) Publication number : **0 498 532 A1**

# EUROPEAN PATENT APPLICATION

(21) Application number : **92300228.1**

(22) Date of filing : **10.01.92**

(51) Int. Cl.⁵ : **A61L 15/16, A61K 37/54, A61K 37/48**

(30) Priority : **10.01.91 US 639477**

(43) Date of publication of application :
**12.08.92 Bulletin 92/33**

(84) Designated Contracting States :
**AT BE CH DE DK ES FR GB GR IT LI LU MC NL PT SE**

(71) Applicant : **E.R. SQUIBB & SONS, INC.**
**Lawrenceville-Princeton Road**
**Princeton New Jersey 08543-4000 (US)**

(72) Inventor : **Constantine, Barry E.**
**163 Van Sant Avenue**
**Island Heights, New Jersey (US)**
Inventor : **Freeman, Frank**
**10 Brandon Road**
**Lawrenceville, New Jersey (US)**
Inventor : **Cilento, Rodolfo D.**
**478 Hobart Street**
**North Brunswick, New Jersey (US)**
Inventor : **Monte, Karen Ann**
**15 Civic Center Drive**
**East Brunswick, New Jersey (US)**

(74) Representative : **Thomas, Roger Tamlyn et al**
**D. Young & Co. 10 Staple Inn**
**London WC1V 7RD (GB)**

(54) **Necrotic tissue debridement powder composition containing a proteolytic enzyme.**

(57) A powder composition useful for debriding wounds comprising an enzyme dispersed in a pharmaceutically acceptable excipient.

EP 0 498 532 A1

## Background of The Invention

The removal of necrotic debris from wounds such as leg ulcers, decubitus ulcers, and burns is important for several reasons. It reduces the potential of infection, promotes wound healing, and influences graft take. Various procedures are available for this purpose including surgical procedures, treatment with antiseptics or antibiotics, and the use of proteolytic enzymes.

Proteolytic enzymes including trypsin, chymotrypsin, papain, ficin, and bromelain as well as subtilisin, which is obtained from the bacteria Bacillus subtilis, have been reported to be useful for the removal of necrotic debris. Noe et al. in United States Patent 3,409,719 describe employing subtilisin derived from the growth of Bacillus subtilis in ointment or gel form for wound debriding.

Flint Laboratories markets Travase® Ointment for wound debridement. It contains subtilisin in a hydrophobic ointment base consisting of 95% white petrolatum and 5% polyethylene. One gram of the ointment contains approximately 82,000 casein units of proteolytic activity. A casein unit is the amount of enzyme required to produce the same optical density at 275 mμ as that of a solution of 1.5 mg. tyrosine/ml. after the enzyme has been incubated with 35 mg. of casein at 37° C. for one minute. In use, body temperature is relied upon to reduce the viscosity of the ointment vehicle to release the subtilisin which is then activated by the moisture in the wound.

Dow B. Hickman, Inc. markets Granulex® for the debridement of eschar and the treatment of wounds. Granulex is in aerosol form and each 0.82 cc. of medication delivered to the wound site contains trypsin crystallized 0.1 mg., Balsam Peru 72.5 mg., castor oil 650 mg., and an emulsifier.

Knoll Pharmaceuticals markets Santyl® Ointment for debriding chronic dermal ulcers and severly burned areas. It is a sterile enzymatic debriding ointment which contains 250 collagenase units per gram of white petrolatum. The enzyme collagenase is derived from the fermentation by Clostridium histolyticum and can digest native and denatured collagen in necrotic tissue.

Parke-Davis markets Elase®, Elase® Ointment, and Elase-Chloromycetin® Ointment. Elase and Elase Ointment are used as topical debriding agents in a variety of inflammatory and infected lesions including surgical wounds, ulcerative lesions, and burns. Elase-Chloromycetin Ointment is indicated for use in the treatment of infected lesions where a debriding agent and a topical antibiotic are desired. Elase is a combination of fibrinolysin and desoxyribonuclease supplied as a lyophilized powder for solution and in an ointment base of liquid petrolatum and polyethylene.

Rystan Company markets Panafil® Ointment for enzymatic debridement of necrotic tissue which contains the proteolytic enzyme papain in a hydrophilic base.

Petereit et al. in United States Patent 4,904,469 describe a wound covering made from a polysaccharide base, particularly cellulose, which is formed as a fabric and contains non-immobilized enzymes such as proteases.

Bolton et al. in United States Patent 4,668,228 disclose a wound debriding tape comprising an enzyme such as subtilisin in dry powdered form on the adhesive mass surface of an occlusive or semi-occlusive adhesive tape.

## Summary Of This Invention

This invention is directed to a necrotic tissue debridement composition in powder form.

## Detailed Description Of Preferred Embodiments

A powder composition is provided containing one or more wound debriding enzymes dispersed in a pharmaceutically acceptable carrier. By employing a powder delivery system, activation of the debriding enzyme by wound fluid occurs more rapidly than in ointment formulations resulting in a more effective debridement process, i.e., the level of enzyme delivered to the wound is less in the powder formulation but produces an equal or greater level of debridement than an ointment particularly when the powder is covered by an occlusive dressing.

Suitable debridement enzymes for use in the powder composition of this invention include proteolytic enzymes such as trypsin, chymptrypsin, papain, ficin, and bromelain as well as subtilisin which is obtained from the bacteria Bacillus subtilis. The debridement enzyme is present in the powder composition at from about 1 mg. (approximately 1,500 proteolytic casein units) to about 20 mg. (approximately 30,000 proteolytic casein units per 100 mg. of powder.

The preferred debriding enzyme is Subtilin A a proteolytic enzyme elaborated by Bacillus subtilis.

The pharmaceutically acceptable excipient which forms the bulk of the powder composition is any nonirritating, nonabsorbing or minimally absorbing, inert carrier material normally utilized in topical pharmaceutical powders. Suitable materials include lactose, mannitol, sorbitol, sucrose, inositol, dextrose, ethyl cellulose, polyethylene glycol having a molecular weight of 1000-8000, etc., with lactose being preferred.

In addition to the debriding enzyme and excipient, the powder can contain other ingredients such as stabilizers, preservatives, etc. Also, a small amount of a pharmaceutically active ingredient such as an antibiotic or wound healing agent can be included within the powder.

The preferred debriding powder compositions of this invention contains from about 2 to about 20 mg. of Subtilisin A per 100 mg. of lactose packaged for a single use. Of course, it is possible to prepare the powder in greater amounts for multiple uses.

EXAMPLE

This example was performed to compare the debridement of the powder formulations of this invention with commercially available Travase® Ointment (Flint Laboratories).

The formulations were applied to premoistened, bilateral, full-thickness scald wounds on anesthetized guinea pigs. Wounds were inflicted using circulating 90°C water for 25 seconds through a latex template with 5 square cm opening which protected the skin surrounding the wound site and also standardized the wound area.

Following application of the formulations to the wound site, a polyurethane film dressing (Bioclusive® of Johnson & Johnson) was applied to contain the enzyme over the wound area. The dressing was secured with elactic tape (Elastikon® of Johnson & Johnson). The subjects were kept undisturbed for 14 hours after which time they were sacrificed. The dressings were removed and the debridement activity was assessed. Debridement activity was expressed in terms of percent of wound area capable of being wiped away with a cotton guaze sponge.

The amount of subtilisin employed in the powder formulation was varied from 2.5 mg. to 20 mg. per 100 mg. of lactose as expressed in Proteolytic Casein Units (PCU).

| Mg. of Subtilisin A in 100 mg. of lactose | PCU/100 mg. of powder | PCU/cm² of wound area | number of wounds | %Debridement |
|---|---|---|---|---|
| 20 | 29,872.6 | 6000 | 6 | 100 |
| 10 | 14,936.3 | 3000 | 6 | 78 |
| 5 | 7,468.2 | 1500 | 11 | 76 |
| 2.5 | 3,734.1 | 750 | 11 | 79 |
| Travase® Ointment 16,400 PCU in 0.2 cc. | | 3280 | 9 | 55 |

## Claims

1. A powder composition for debriding wounds comprising a pharmaceutically acceptable excipient and a wound debriding enzyme wherein the enzyme is present at from about 1500 to about 30,000 proteolytic casein units per 100 mg. of powder.

2. The composition of Claim 1 wherein the enzyme is a proteolytic enzyme selected from trypsin, chymotrypain, papain, ficin, and bromelain.

3. The composition of Claim 1 wherein the enzyme is a subtilisin obtained from the bacteria Bacillus subtilis.

4. The composition of any preceding claim wherein the powder, in addition, contains one or more stabilizers, preservatives or a pharmaceutically active ingredient.

5. The composition of any preceding claim wherein the excipient is lactose.

6. The composition of Claim 5 wherein the enzyme is Subtilisin A.

7. A powder composition for wound debridement in unit dosage form comprising from about 2 to about 20 mg. of subtilin A per 100 mg. of lactose therein.

## Claims for the following Contracting States : ES, GR

1. A process for preparing a powder composition for debriding wounds comprising a pharmaceutically acceptable excipient and a wound debriding enzyme wherein the enzyme is present at from about 1500 to about 30,000 proteolytic casein units per 100 mg. of powder said process comprising preparing a mixture of the excipient and the enzyme in powder form.

2. The process of Claim 1 wherein the enzyme is a proteolytic enzyme selected from trypsin, chymotrypain, papain, ficin, and bromelain.

3. The process of Claim 1 wherein the enzyme is a subtilisin obtained from the bacteria Bacillus subtilis.

4. The process of any preceding claim wherein the powder, in addition, contains one or more stabilizers, preservatives, or a pharmaceutically active ingredient.

5. The process of any preceding claim wherein the excipient is lactose.

6. The process of Claim 5 wherein the enzyme is Subtilisin A.

7. A process for preparing a powder composition for wound debridement in unit dosage form comprising from about 2 to about 20 mg. of Subtilin A per 100 mg. of lactose therein, said process comprising preparing a mixture of the lactose and the Subtilin A in powder form.

EP 0 498 532 A1

# EUROPEAN SEARCH REPORT

**European Patent Office**

Application Number

EP 92 30 0228

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 296 787 (JOHNSON & JOHNSON)<br>* page 4, line 40 - page 5, line 35 *<br>--- | 1-4 | A61L15/16<br>A61K37/54<br>A61K37/48 |
| X<br><br>D | EP-A-0 194 647 (JOHNSON & JOHNSON)<br>* page 4, line 10 - line 26 *<br>* page 5, line 11 - line 20 *<br>& US-A-4 668 228<br>--- | 1-4 | |
| X | WO-A-8 402 274 (HAFSTEN R.)<br>* page 12 - page 13; example 1 *<br>* claims 3-4 *<br>--- | 1-4 | |
| A | FR-A-2 552 667 (CESKOSLOVENSKA AKADEMIE VED)<br>* claims 1-2 *<br>--- | 1-7 | |
| A | FR-A-2 558 171 (L'OREAL)<br>* page 3, line 16 - line 26 *<br>--- | 1-7 | |
| X,P | WO-A-9 117 255 (UNIVERSITY OF IOWA RESEARCH FOUNDATION)<br>* claims 2,4-5 *<br>--- | 1-7 | **TECHNICAL FIELDS SEARCHED (Int. Cl.5)** |
| A,D | US-A-3 409 719 (NOE A. F. ET AL)<br>* the whole document *<br>----- | 1-7 | A61L<br>A61K |

The present search report has been drawn up for all claims

| Place of search | Date of completion of the search | Examiner |
|---|---|---|
| BERLIN | 13 MAY 1992 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)